# EUROPÄISCHE PATENTSCHRIFT

(11) **EP 0 536 595 B1**
(45) Veröffentlichungstag und Bekanntmachung des Hinweises auf die Patenterteilung: **05.07.1995**
(21) Anmeldenummer: 92116324.2
(22) Anmeldetag: 24.09.1992
(51) Int. Cl.: A61K 9/28, A61K 9/32, A61K 9/36

(54) **Verfahren zum Überziehen von Arzneiformen**
Process for coating pharmaceutical preparations
Procédé pour enrober des formes pharmaceutiques

(30) Priorität: 07.10.1991 DE 4133192
(43) Veröffentlichungstag der Anmeldung: 14.04.1993
(73) Patentinhaber: BASF Aktiengesellschaft, 67063 Ludwigshafen (DE)
(72) Erfinder: Grabowski, Sven, Dr., W-6700 Ludwigshafen (DE); Wendel, Kurt, Dr., W-6700 Ludwigshafen (DE); Kah-Helbig, Astrid, Dr., W-6730 Neustadt (DE)

(56) Entgegenhaltungen:
- EP-A- 0 408 099
- BIOMATERIALS Bd. 11, Nr. 5, Juli 1990, GUILDFORD, SURREY, GB Seiten 345 - 350 ,XP000140305 J. HELLER, S. H. PANGBURN ET AL.

## Beschreibung

### Gebiet der Erfindung

Die Erfindung betrifft ein Verfahren zum Überziehen von Arzneiformen mit einem in Wasser dispergierten Überzugsmittel, sowie das pulverförmige, redispergierbare Überzugsmittel.

### Stand der Technik

DE-C 21 35 073 beschreibt das Überziehen von Arzneiformen mittels einer wäßrigen Dispersion eines aus Vinylmonomeren gebildeten Polymeren, das zu 10 bis 55 Gew.-% aus Monomeren mit einer Carboxyl- oder Aminogruppe aufgebaut ist. Die Beschichtung mit wäßrigen Überzugsmitteldispersionen hat vor der Anwendung organischer Überzugsmittellösungen den Vorteil, daß die mit den organischen Lösungsmitteln verbundene Feuergefahr und Umweltbelastung vermieden wird, aber den Nachteil, daß die Herstellung der wäßrigen Überzugsdispersionen schwierig und von dem Arzneihersteller nicht zu bewerkstelligen ist. Er muß daher das Bindemittel zusammen mit dem Wasser beziehen und lagern. Dagegen kann er organische Lösungen aus dem pulverförmigen Überzugsmittel ohne Schwierigkeiten selbst herstellen.

Nach der DE-C 25 12 238 lassen sich zwar wäßrige Dispersionen von Bindemitteln für Arzneimittelüberzüge durch Sprühtrocknen in ein Pulver überführen, jedoch muß dieses zur Anwendung in einem organischen Lösungsmittel gelöst werden.

Nach der DE-A 30 49 179 erzeugt man Arzneimittelüberzüge aus einem sprühgetrockneten Emulsionspolymerisat-Pulver, indem man es in der wäßrigen Lösung eines nicht-flüchtigen Weichmachungsmittels suspendiert und die Suspension auf Arzneiformen aufträgt und erwärmt, wodurch ein Teil des Wassers verdampft und das suspendierte Überzugsmittelpulver in dem Weichmacher in Lösung geht. Diese Lösung fließt zu einer Überzugsschicht zusammen und erstarrt beim Erkalten. Dieses Verfahren wird als "Thermogelierung" bezeichnet. Der Filmbildungsvorgang unterscheidet sich wesentlich von dem echter wäßriger Latices. Dort ist der Kohäsionsdruck zwischen den Latexteilchen beim allmählichen Verdunsten des Wassers entscheidend, d.h. die Latexteilchen gehen unmittelbar als solche in den zusammenhängenden Film über und nicht über die Zwischenstufe einer Lösung.

Das Thermogelierungsverfahren erfüllt nicht immer alle Forderungen, die an ein Überzugsverfahren für Arzneiformen und an die dafür eingesetzten Überzugsmittel gestellt werden. Da das Überzugsmittelpulver nicht als Latex redispergiert, sondern in größeren Partikeln suspendiert wird, ist die entstehende Suspension oft nicht über ausreichend lange Zeit lagerfähig. Um einen glatten und vollkommen porenfreien Überzug zu erhalten, müssen erhebliche Mengen an Weichmachern und hohe Gelierungstemperaturen angewendet werden, andernfalls entstehen rauhe, mehr oder weniger poröse Über-Züge, oder es müssen sehr dicke Schichten aufgetragen werden.

In der DE-A 34 38 291 wird ein Verfahren zur Herstellung einer wäßrigen Überzugsdispersion und ihre Verwendung zum Überziehen von Arzneimitteln beschrieben. Ein pulverförmiges, in Wasser quellbares, aber nicht lösliches Mischpolymerisat aus 5 bis 20 Gew.-% einer monoethylenisch ungesättigten, radikalisch polymerisierbaren quartären Ammoniumverbindung und 95 bis 70 Gew.-% wenigstens eines nichtionischen, wasserunlösliche Homopolymerisate bildenden Comonomeren kann bei erhöhter Temperatur (50 bis 100°C, vorzugsweise 60 bis 80°C) und wenigstens 15 Minuten Rühren in Wasser dispergiert werden. Aus dieser Dispersion lassen sich pharmazeutische Überzüge oder Filme mit einer vom pH-Wert des umgebenden Mediums unabhängigen Diffusionsdurchlässigkeit herstellen.

Das entsprechende Handelsprodukt, ein Terpolymerisat aus Methylmethacrylat, Ethylacrylat und Trimethylammonioethylmethacrylat-chlorid, kann als granuliertes Substanzpolymerisat bezogen und entsprechend der DE-A 34 38 291 nach Mahlen zu feinem Pulver in Wasser bei erhöhter Temperatur unter Rühren redispergiert werden, gegebenenfalls unter Zusatz von Weichmachern. Dieses aufwendige Verfahren wird jedoch im allgemeinen nicht vom Arzneihersteller durchgeführt.

Demgegenüber ist ein redispergierbares Dispersionspulver, das erst kurz vor dem Einsatz zum Überziehen von Arzneiformen zusammen mit den übrigen Zusätzen in ein wäßriges Überzugsmittel überführt werden kann, ein großer Vorteil. In der EP-C 88 951 ist ein derartiges trockenes Pulver, bestehend aus einem Emulsionspolymerisat aus
A) 20 bis 85 Gew.-% Einheiten wenigstens eines Alkylesters der Acryl- und/oder Methacrylsäure,
B) 80 bis 15 Gew.-% Einheiten wenigstens eines zur Salzbildung befähigten, radikalisch polymerisierbaren Monomeren und
C) gegebenenfalls bis zu 30 Gew.-%, bezogen auf die Summe von A + B, Einheiten von einem oder mehreren anderen radikalisch polymerisierbaren Monomeren
beschrieben, dessen Pulverkörnchen aus lose aggregierten Feinpartikeln aufgebaut sind.

Dieses Pulver kann in eine Überzugsmittel-Dispersion überführt werden, indem man es in Wasser einträgt und in Gegenwart einer Menge eines zur Salzbildung mit den Monomereneinheiten B) befähigten Mittels, die so bemessen ist, daß nach ihrer Umsetzung mit den Monomereneinheiten B) insgesamt 10 bis 12 Gew.-%, bezogen auf das Gewicht des Mischpolymerisats dieser Einheiten, in der Salzform vorliegen, zu wenigstens 80 Gew.-% zu Latexteilchen redispergiert.

Je nach der sauren oder basischen Natur der salzbildenden Gruppen in dem Emulsionspolymerisat entstehen magensaftresistente und darmsaftlösliche oder magensaftlösliche Überzüge. Magensaftresistente Überzüge werden erhalten, wenn die Monomeren der Komponente B Carboxyl- bzw. Carboxylatgruppen enthalten. Bei hohen Gehalten an diesen Gruppen sind die Überzüge schnell im alkalischen Darmsaft löslich, bei geringeren Gehalten lösen sie sich langsamer oder werden durch Quellung diffusionsdurchlässig. Aminogruppenhaltige Monomere machen den Überzug bei hohen Anteilen magensaftlöslich, bei niedrigen Anteilen in Magensaft quellbar und diffusionsdurchlässig.

Bedingt durch die zur Redispergierung des vorbeschriebenen Dispersionspulvers notwendigen salzbildenden Monomereinheiten ist die Löslichkeit bzw. Diffusionsdurchlässigkeit des Überzuges abhängig vom pH-Wert. Ein in Wasser wie auch in den Verdauungssäften (Magensaft und Darmsaft) unlöslicher, jedoch quellfähiger und permeabler Filmbildner für Überzüge und/oder Matrixtabletten, der die Freisetzung der aktiven Arzneisubstanz über die diffusionskontrollierende Filmdicke entsprechend den pharmakokinetischen und therapeutischen Ansprüchen pH-unabhängig zu regulieren gestattet, kann nach diesem Verfahren nicht realisiert werden.

Darüber hinaus muß darauf geachtet werden, daß die Monomerenzusammensetzung des Filmbildners derart gewählt wird, daß eine Sprühtrocknung oder Gefriertrocknung der Dispersion durchführbar ist und zudem das Emulsionspolymerisat in Pulverform bei Raumtemperatur im unverglasten Zustand gelagert werden kann. Daher ist eine Glastemperatur des Mischpolymerisats von oberhalb 25°C, vorzugsweise oberhalb 40° erforderlich. Mit der Glastemperatur steigt aber die Härte und Sprödigkeit des Films. Folglich müssen zur Verarbeitung des Überzugsmittels Weichmacher eingesetzt werden, wodurch die Glastemperatur aber wieder sinkt.

### Aufgabe und Lösung

Der Erfindung lag die Aufgabe zugrunde, die Vorteile der Arzneiformbeschichtung mit einer Überzugsmitteldispersion mit denen des Einsatzes von trockenen Überzugsmittelpulvern zu verbinden. Es war ein Verfahren zu entwickeln, das keine großen Weichmachermengen und keine hohen Filmbildungstemperaturen erfordert, aber glatte und porenfreie Überzüge bildet. Das Dispersionspulver (das getrocknete Emulsionspolymerisat) sollte sich ohne Zusatz von salzbildenden Mitteln und von Weichmachern in kaltem Wasser ohne längeres Rühren problemlos redispergieren lassen. Weiterhin sollte vor allem das Pulver, aber auch dessen wäßrige Redispersion längere Zeit lagerfähig sein. Der aus diesem Überzugsmittel resultierende Film sollte eine pH-unabhängige Freisetzung des Arzneistoffes ermöglichen, wobei einerseits eine Instant-Release-Freisetzung aus den mit dem Überzugsmittel überzogenen Arzneiformen angestrebt war, und andererseits auch eine retardierte Freisetzung aus Matrixtabletten, die durch Feuchtgranulation mit dem Überzugsmittel und anschließend Tablettierung erhalten wurden.

Diese Aufgabe wird durch das Verfahren gemäß dem Anspruch 1 gelöst.

### Das Emulsionspolymerisat

Zur Lösung der beschriebenen Aufgabe geht die Erfindung von wäßrigen Polymerisatdispersionen aus, die durch Emulsionspolymerisation ethylenisch ungesättigter Verbindungen mittels freie Radikale liefernder Initiatoren in Gegenwart von verzuckerter Stärke vom gewichtsmittleren Molekulargewicht 2 500 bis 25 000 und gegebenenfalls weiterer üblicher Zusatzstoffe, wie z.B. in der DE-A 39 22 784 beschrieben, bei 30 bis 120, vorzugsweise 40 bis 100°C hergestellt werden können.

Geeignete ethylenisch ungesättigte, polymerisierbare Monomere sind z.B. (Meth-)Acrylsäureester von C₁- bis C₁₈-Alkoholen, wie Methylmethacrylat und Ethylacrylat, auch Hydroxyalkylester der (Meth-)Acrylsäure, Vinylester und Vinyllactame; ferner ungesättigte Mono- oder Dicarbonsäuren wie (Meth-)Acrylsäure, Malein-, Fumar-, Croton- und Itakonsäure sowie Halbester oder Halbamide dieser Säuren. Geeignete Monomere mit Aminogruppen sind Vinylimidazol, Vinylimidazolin, Vinylimidazolidin, Vinylpyridin, Monoalkyl- und Dialkylaminoalkylester oder Monoalkyl- oder Dialkylaminoalkylamide von ungesättigten polymerisierbaren Carbonsäuren. Ebenfalls eingesetzt werden können anionische Monomere wie Salze von Acrylamidoalkylsulfonsäuren, kationische Monomere wie Trimethylammonioethylmethacrylat-chlorid, multifunktionelle, vernetzende Monomere wie Methylol(meth)acrylamide und deren Derivate.

Die Auswahl der Monomeren bzw. Monomergemische sowie deren Verhältnis zur Menge des eingesetzten Stärkederivates richtet sich einerseits nach den Erfordernissen des Überzugsverfahrens (Glastemperatur, Mindestfilmbildetemperatur), andererseits nach den gewünschten Eigenschaften des Überzugs (Löslichkeitsverhalten in verschiedenen Medien, Härte, Sprödigkeit und Elastizität des Filmes).

Die einzusetzende Menge an verzuckerter Stärke beträgt 10 bis 200, vorzugsweise 20 bis 50 Gew.-%, bezogen auf das Polymerisat. Verzuckerte Stärke besteht aus durch Hydrolyse in wäßriger Phase erhaltenen Stärkeabbauprodukten eines gewichtsmittleren Molekulargewichtes von 2500 bis 25000. Sie sind als solche im Handel erhältlich (z.B. die C* PUR Produkte 01906, 01908, 01910, 01912, 01915, 01921, 01924, 01932 oder 01934 der Cerestar Deutschland GmbH, D-4150 Krefeld 12).

Die Herstellung verzuckerter Stärken ist allgemein bekannt und u.a. in Günther Tegge, Stärke und Stärkederivate, Behr's Verlag, Hamburg 1984, S. 173 und S. 220 ff sowie in der EP-A 441 197 beschrieben. Besonders geeignet sind z.B. die den "Richtlinien für Stärke und bestimmte Stärkeerzeugnisse" von 1975 des Bundes für Lebensmittelrecht und Lebensmittelkunde e.V. entsprechenden Stärkeverzuckerungsprodukte, die den rechtlichen Status eines Lebensmittels haben. Vorzugsweise handelt es sich bei den erfindungsgemäß zu verwendenden verzuckerten Stärken um solche, deren gewichtsmittleres Molekulargewicht M_{w} im Bereich von 4000 bis 16000, besonders bevorzugt im Bereich von 6500 bis 13000 liegt.

Die erfindungsgemäß zu verwendenden verzuckerten Stärken sind normalerweise bei Raumtemperatur in Wasser vollständig löslich, wobei die Löslichkeitsgrenze in der Regel oberhalb von 50 Gew.-% liegt, was sich für die Herstellung der erfindungsgemäßen wäßrigen Polymerisatdispersionen als besonders vorteilhaft erweist.

Es hat sich ferner als günstig erwiesen, wenn die erfindungsgemäß zu verwendenden verzuckerten Stärken eine Unheitlichkeit U (definiert als Verhältnis von gewichtsmittlerem Molekulargewicht M_{w} zu zahlenmittlerem Molekulargewicht Mₙ; U charakterisiert die Molekulargewichtsverteilung) im Bereich von 6 bis 12 aufweisen. Besonders vorteilhaft beträgt U 7 bis 11 und ganz besonders günstig ist ein U von 8 bis 10.

Ferner ist es von Vorteil, wenn der Gewichtsanteil der erfindungsgemäß zu verwendenden verzuckerten Stärken, der ein Molekulargewicht unterhalb von 1000 aufweist, wenigstens 10 Gew.-%, jedoch nicht mehr als 70 Gew.-% beträgt. Besonders bevorzugt liegt dieser Gewichtsanteil im Bereich von 20 bis 40 Gew.-%.

Darüber hinaus ist es empfehlenswert, solche erfindungsgemäß zu verwendenden verzuckerten Stärken anzuwenden, deren Dextroseequivalent DE 5 bis 40, vorzugsweise 10 bis 30 und besonders bevorzugt 10 bis 20 beträgt. Der DE-Wert charakterisiert das Reduktionsvermögen bezogen auf das Reduktionsvermögen von wasserfreier Dextrose und wird nach DIN 10308 Ausgabe 5.71, des Normenausschusses Lebensmittel und landwirtschaftliche Produkte, bestimmt (vgl. auch Günther Tegge, Stärke und Stärkederivate, Behr's Verlag, Hamburg 1984, S. 305).

Außerdem hat es sich gezeigt, daß in ihrem Eigenschaftsprofil besonders günstige wäßrige Polymerisatdispersionen dann erhalten werden, wenn man erfindungsgemäß zu verwendende verzuckerte Stärken einsetzt, deren 40 gew.-%igen wäßrigen Lösungen bei 25°C und einem Schergefälle von 75 s⁻¹ eine nach DIN 53 019 bestimmte dynamische Viskosität η⁴⁰ (Pa·s] von 0,01 bis 0,06, vorzugsweise von 0,015 bis 0,04 und besonders bevorzugt von 0,02 bis 0,035 aufweisen.

An dieser Stelle sei festgehalten, daß in dieser Schrift, sofern nichts anderes erwähnt ist, Aussagen über das Molekulargewicht von erfindungsgemäß zu verwendenden verzuckerten Stärken auf Bestimmungen mittels der Gelpermeationschromatographie beruhen, wobei unter folgenden Bedingungen chromatographiert wurde.
- Säulen:: 3 Stück 7.5 x 600 mm Stahl gefüllt mit TSK-Gel G 2000 PW; G 3000 PW u. G 4000 PW. Porenw. 5 »m
- Eluent:: Wasser dest.
- Temp.:: RT
- Detektion:: Differentialrefraktometer (z.B. ERC 7511)
- Fluss:: 0.8 ml/min. Pumpe: (z.B. ERC 64.00)
- Injectv.:: 20 »l Ventil: (z.B. VICI 6-Wege-Ventil)
- Auswertung:: Bruker Chromstar GPC-Software
- Eichung:: Die Eichung erfolgte im niedermolekularen Bereich mit Glucose, Raffinose, Maltose und Maltopentose. Für den höhermolekularen Bereich wurden Pullulan-Standards mit einer Polydispersität < 1.2 verwendet.

Als Ausgangsstärken zur Herstellung der erfindungsgemäß zu verwendenden verzuckerten Stärken sind prinzipiell alle nativen Stärken wie Getreidestärken (z.B. Mais, Weizen, Reis oder Hirse), Knollen- und Wurzelstärken (z.B. Kartoffeln, Tapiokawurzeln oder Arrowroot) oder Sagostärken geeignet.

Ein wesentlicher Vorzug der erfindungsgemäß zu verwendenden verzuckerten Stärken ist, daß es hinsichtlich ihrer Anwendung, abgesehen von der in einfachster Weise durchzuführenden partiellen Hydrolyse der Ausgangsstärke zu ihrer Herstellung, keiner weiteren chemischen Modifizierung bedarf. Selbstverständlich können sie aber auch in z.B. durch Veretherung oder Veresterung chemisch modifizierter Form erfindungsgemäß angewendet werden. Diese chemische Modifizierung kann auch bereits an der Ausgangsstärke vor deren Abbau durchgeführt worden sein. Veresterungen sind sowohl mit anorganischen als auch mit organischen Säuren, deren Anhydriden oder Chloriden möglich. Von besonderem Interesse sind phosphatierte und acetylierte abgebaute Stärken. Die gängigste Methode zur Veretherung ist die Behandlung mit organischen Halogenverbindungen, Epoxiden oder Sulfaten in wäßriger alkalischer Lösung. Besonders geeignete Ether sind Alkylether, Hydroxy- alkylether, Carboxyalkylether und Allylether. Ferner kommen auch Umsetzungsprodukte mit 2,3-Epoxipropyltrimethylammoniumchlorid in Betracht. Chemisch nicht modifizierte verzuckerte Stärken sind bevorzugt.

Als freie Radikale bildende Initiatoren können Wasserstoffperoxid, wasserlösliche organische Peroxide und Hydroperoxide, gegebenenfalls in Kombination mit reduzierenden Verbindungen wie Ascorbinsäure, wasserlösliche Azoverbindungen wie 2,2ʼ-Azobis(2-amidinopropan)-dihydrochlorid, ferner anorganische Peroxide, wie Alkalimetall- oder Ammoniumsalze der Peroxodischwefelsäure, in Mengen von 0,1 bis 2, vorzugsweise 0,2 bis 1 Gew.-%, bezogen auf die Gesamtmenge der Monomeren, verwendet werden.

Bei Bedarf können dem Reaktionsgemisch weitere übliche Hilfsstoffe zugesetzt werden. Zu diesen Hilfsstoffen zählen Keimlatices, welche die Reproduzierbarkeit der Teilchengröße der Endprodukte verbessern, sowie Puffergemische, Komplexbildner, Dispergiermittel und Emulgatoren. Das Emulsionspolymerisat wird in der Regel in Gegenwart geringer Mengen (ca. 0,1 bis 5, vorzugsweise 0,5 bis 1,5 Gew.-%, bezogen auf die Monomeren), an anionischen, kationischen oder nichtionischen Tensiden (Emulgatoren) oder deren verträglichen Gemischen in Form eines wäßrigen Latex mit einem Feststoffgehalt von 30 bis 70 Gew.-% hergestellt. Bei der Gewinnung des trockenen Polymerisatpulvers aus dem Latex ist darauf zu achten, daß die Latexteilchen als solche erhalten bleiben und nicht zu unzertrennbaren Aggregaten verkleben. Das gelingt, wenn bei der Isolierung des Polymerisats Temperaturen oberhalb der Mindestfilmbildetemperatur vermieden werden. Geeignet sind vor allem die Verfahren der Sprühtrocknung und der Gefriertrocknung. Die Herstellung des pulverförmigen Emulsionspolymerisats ist nicht Gegenstand der Erfindung.

### Das Überzugsmittel und dessen Anwendung

Das Überzugsmittel besteht aus der 0 bis 5, vorzugsweise 0 bis 1 Gew.-% Tensid enthaltendenden wäßrigen Redispersion des pulverförmigen Emulsionspolymerisates mit einem Festgehalt von 10 bis 60, vorzugsweise 20 bis 45 Gew.-%. Es enthält neben dem Polymerisat 0 bis 90, vorzugsweise 20 bis 70 Gew.%, bezogen auf den Gesamtfestgehalt, übliche galenische Hilfsmittel, wie Pigmente, Gleitmittel wie Magnesiumstearat oder Talkum, Geschmacksstoffe, Zucker, Glanzmittel, Weichmacher usw., die üblicherweise getrennt in Wasser dispergiert und später zugemischt oder direkt in die Redispersion eingearbeitet werden. Der Bindemittel-Anteil liegt bei 10 bis 100, vorzugsweise zwischen 30 und 90 Gew.-%, bezogen auf die Gesamtfeststoffe. Feststoffgehalt und Viskosität des Überzugsmittels richten sich nach dem Auftragsverfahren. Im allgemeinen beträgt die Viskosität etwa 5 bis 40 mPas. Es können alle gebräuchlichen Auftragsverfahren angewendet werden, z.B. die übliche Kesseldragierung oder die Beschichtung von Arzneiformen in einer Wirbelschichtapparatur.

Man trägt das Überzugsmittel in der Regel in einer Menge von 0,5 bis 10 mg Feststoff je Quadratzentimeter der Arzneioberfläche auf und erhält Schichtdicken von 1 bis 100 »m (Mikron). Das Überzugsmittel kann - wie bei anderen Überzugsverfahren - portionsweise in mehreren Schichten aufgetragen werden, zwischen denen jeweils getrocknet werden kann. Ebenso kann man das Überzugsmittel kontinuierlich auftragen und gleichzeitig mittels leicht erwärmter Luft trocknen. Es genügt im allgemeinen, an der Oberfläche der zu überziehenden Arzneiform höchstens ca. 40°C, meistens sogar unter 35°C, vorzugsweise 25 bis 35°C zu erzeugen, wobei je nach Verdunstungsgeschwindigkeit und Gerätekonstruktion Zulufttemperaturen von 40 bis 70°C erforderlich sind.

Als Bindemittel wird die filmbildende Komponente (das Polymer) des Überzugsmittels bezeichnet, unabhängig davon, ob sie gemeinsam mit ungelösten Hilfsstoffen angewendet wird. Dementsprechend kann die Filmbildung entweder in Abwesenheit von ungelösten Zusätzen zu einem mehr oder weniger klaren Polymerisatüberzug führen oder in Anwesenheit von ungelösten Zusätzen zu einem deckenden Überzug. Die Filmbildung tritt unmittelbar aus dem Latexzustand ein. In dem eingesetzten pulverförmigen Emulsionspolymerisat sind die Latexteilchen in ihrer ursprünglichen Gestalt vorhanden, wenn auch zu lokkeren Aggregaten zusammengeballt. Sie können daher nahezu vollständig zu einzelnen dispergierten Latexteilchen verteilt werden.

Das Bindemittel zur Bereitung eines wäßrigen Überzugsmittels kann bis zur Herstellung des Überzugsmittels raum- und gewichtssparend und ohne Gefahr eines Qualitätsverlustes als trockenes Pulver transportiert und gelagert werden. Bei der Redispergierung sind weder längeres Rühren noch erhöhte Temperatur noch Zusätze von salzbildenden Mitteln oder Weichmachern erforderlich.

Durch die geringe Menge an erforderlichem Tensid bei der Emulsionspolymerisation ist sowohl die Primärdispersion als auch die aus der Redispergierung des Dispersionspulvers resultierende Überzugsmitteldispersion schaumarm. Trotzdem ist sie ausreichend stabil, beispielsweise gegen Kälte, Hitze und Elektrolytzusatz.

Das erfindungsgemäß einzusetzende redispergierbare Dispersionspulver kann durch Gefriertrocknung wie auch durch Sprühtrocknung des Emulsionspolymerisates hergestellt werden. Es lassen sich auch redispergierbare Pulver aus Mischpolymerisaten mit einer Glastemperatur des reinen Copolymerisats unter 25°C und einer entsprechend niedrigen, unterhalb der Raumtemperatur liegenden Mindestfilmbildetemperatur herstellen. Hierfür ist kein Zusatz von Sprühhilfsmitteln bei der Sprühtrocknung erforderlich. Die Lagerstabilität des Dispersionspulvers ist auch ohne Zusätze von Antiblockmitteln gegeben.

Obwohl das Überzugsmittel zur Einstellung der gewünschten Filmhärte und -elastizität auch übliche Weichmachungsmittel enthalten kann, werden davon keine Mengen benötigt, die den Film während der Herstellung weich und klebrig machen, wie während der Filmbildung aus organischen Lösungsmitteln oder bei der Thermogelierung. Weiterhin ist es überraschenderweise möglich, auch von per se sehr weichen Copolymerisaten nicht nur ein redispergierbares, lagerfähiges Dispersionspulver, sondern hieraus dann auch klebfreie, elastische Überzüge mit hoher Reißfestigkeit ohne Zusätze von Weichmachern herzustellen. Dies ist ein wesentlicher Vorzug des erfindungsgemäßen Verfahrens vor der Herstellung von Überzügen aus Copolymerisaten mit zur Sprühtrocknung hinreichend hohen Glastemperaturen (und entsprechend hohen Mindestfilmbildetemperaturen). Ferner lassen sich erfindungsgemäß überraschenderweise trotz des Gehaltes an löslicher verzuckerter Stärke Retardtabletten herstellen.

### Anwendungsgebiete

Je nach der Menge der in der Emulsionspolymerisation eingesetzten verzuckerten Stärke lassen sich die Lösungseigenschaften des resultierenden Filmes steuern. Da prinzipiell - im Gegensatz zur oben zitierten EP-C 88 951 - ionisierbare Gruppen, wie Amino- oder Carboxylgruppen, enthaltende Monomereinheiten im Copolymerisat zur Herstellung der Redispergierfähigkeit zwar einsetzbar, aber nicht erforderlich sind, lassen sich Filme mit einer vom pH-Wert des umgebenden Mediums unabhängigen Diffusionsdurchlässigkeit herstellen. So können nach Feuchtgranulation mit den erfindungsgemäßen Dispersionen und anschließender Tablettierung Matrixtabletten hergestellt werden, die eine retardierte Freisetzung der Arzneisubstanz ermöglichen. Durch Variation des Anteils an verzuckerter Stärke in der Dispersion können aber ebenso Überzüge realisiert werden, die in einem Medium mehr oder weniger rasch und pH-unabhängig zerfallen.

Mit "Arzneiformen" sind alle festen Arzneiformen gemeint, also z.B. Tabletten, Filmtabletten, Dragees, Granulate, jedoch keine Pulver.

### Beispiele

### Beispiel 1

### Herstellung von Retardmatrixtabletten

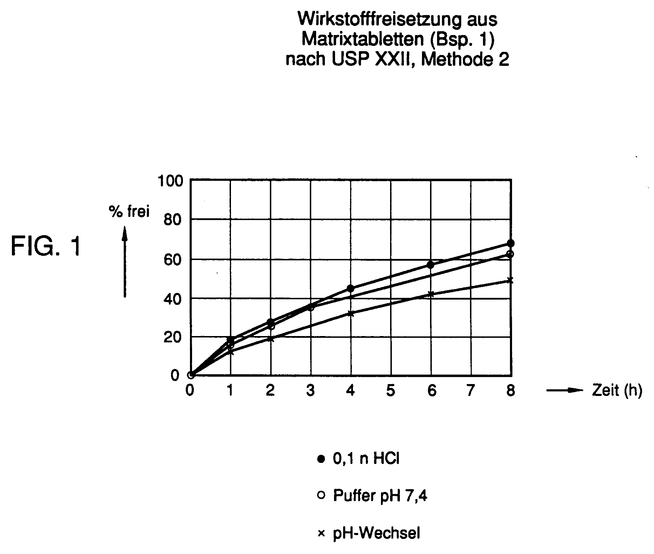

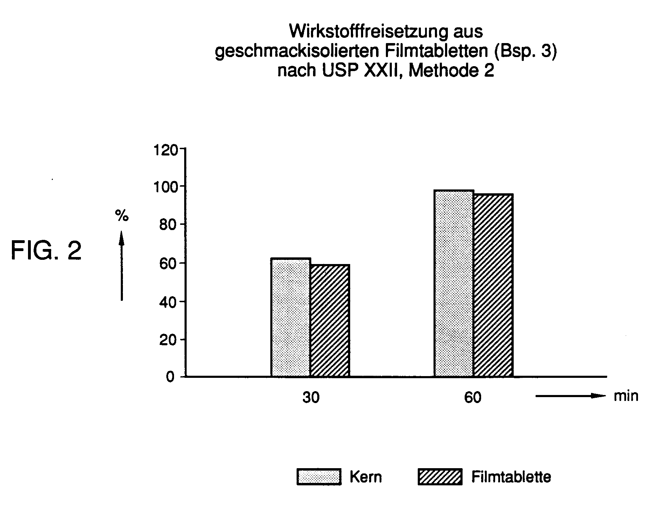

125 g wasserfreies Theophyllin von der Teilchengröße 0,2 bis 0,7 mm wurden mit 75 g Calciumhydrogenphosphat gemischt, mit 20,8 g Wasser befeuchtet und mit 35,23 g einer Polymerdispersion mit 30 % Festgehalt aus Ethylacrylat und Methylmethacrylat im Gewichts-Verhältnis 2:1, die durch Emulsionspolymerisation in Gegenwart von 20 Gew.-% Maltodextrin, bezogen auf die Summe der Monomeren, hergestellt worden war, granuliert. Das Granulat wurde 24 Stunden im Umlufttrockenschrank getrocknet, gesiebt, und auf einem Rundläufer mit einer Preßkraft von 15 kN zu Tabletten mit 8 mm Durchmesser und 215 mg Gewicht verpreßt. Die Tabletten zeigten eine pH-unabhängige, über 8 Std. verzögerte Wirkstofffreisetzung (USP XXII, Methode 2; Abb. 1).

### Beispiel 2

### Anwendung als schnell zerfallender Filmüberzug

In 107,5 g Wasser wurden 1,5 g Sicopharm-Chinolingelblack, 6,0 g Titandioxid und 7,5 g Talkum mittels Ultra-Turrax fein suspendiert. In diese Suspension wurden 125,0 g einer wäßrigen 30%igen Polymerdispersion aus Ethylacrylat und Methylmethacrylat im Gewichts-Verhältnis 2:1, die durch Emulsionspolymerisation in Gegenwart von 30 Gew.-% Maltodextrin, bezogen auf die Summe der Monomeren, Sprühtrocknen und Redispergieren hergestellt worden war, eingerührt. 500 g der so erhaltenen Überzugssuspension wurden auf 2,5 kg Placebo-Kerne in der Wirbelschicht aufgesprüht, entsprechend einer Auftragsmenge von 1,4 mg/cm², berechnet auf das Polymere. Die Zulufttemperatur betrug 42°C, die Sprühgeschwindigkeit ca. 20 g/min. Die überzogenen Tabletten wurden über Nacht im Umlufttrockenschrank bei 40°C getrocknet. Der Überzug war gleichmäßig deckend, glatt und glänzend. Die Zerfallszeit der Tabletten wurde durch das Überziehen praktisch nicht verlängert, sie betrug 3 bis 4 Minuten.

### Beispiel 3

### Anwendung als geschmacksisolierender Überzug

500 g einer 40%igen Sprühsuspension, hergestellt analog Beispiel 2, wobei jedoch bei der Emulsionspolymerisation nur 20 %Maltodextrin (statt 30) eingesetzt worden waren, wurden in einem Accela-Cota (Manesty Machines Ltd., Speke, Liverpool L 249 LQ, UK) auf 5 kg theophyllinhaltiger Kerne mit einer Sprühgeschwindigkeit von ca. 20 g/min. bei einer Zulufttemperatur von 50°C aufgebracht. Die Auftragsmenge betrug 0,7 mg/cm², berechnet auf das Polymere. Die überzogenen Tabletten blieben mehrere Minuten geschmacksisoliert. Die Freisetzung des Wirkstoffs in vitro wurde dadurch nicht verzögert (USP XXII, Methode 2, 0,1 N HCl; ABB: 2).

## Patentansprüche

1. Verfahren zum Überziehen von Arzneiformen durch Auftragen einer wäßrigen Dispersion, die als Bindemittel einen redispergierten Latex eines physiologisch unbedenklichen Emulsionspolymerisates enthält, auf die Arzneiform und Verdunsten des Wassers bei einer Temperatur, bei der die Latexteilchen verfilmen, dadurch gekennzeichnet, daß eine Polymerisatdispersion eingesetzt wird, die durch Emulsionspolymerisation von einem Gewichtsteil wenigstens eines ethylenisch ungesättigten, polymerisierbaren Monomeren mittels freie Radikale liefernder Initiatoren in Gegenwart von 0,1 bis 2 Gew.-Teilen verzuckerter Stärke vom mittleren Molgewicht 2 500 bis 25 000, 0 bis 5 Gew.-%, bezogen auf das Monomere oder die Monomeren, eines Tensids und gegebenenfalls weiterer üblicher Zusatzstoffe, Trocknen zu Pulver und Redispergieren in Wasser hergestellt wurde.

2. Pulverförmiges, in kaltem Wasser ohne weitere Hilfsmittel redispergierbares Gemisch zur Herstellung eines wäßrigen Überzugsmittels für Arzneiformen, dadurch gekennzeichnet, daß es 10 bis 100 Gew.-% eines Emulsionspolymerisates aus 1 Gew.-Teil wenigstens eines ethylenisch ungesättigten, polymerisierbaren Monomeren enthält, das in Gegenwart von 0,1 bis 2 Gew.-Teilen verzuckerter Stärke vom mittleren Molgewicht 2 500 bis 25 000 polymerisiert wurde.

3. Pulverförmiges Gemisch gemäß Anspruch 2, dadurch gekennzeichnet, daß das Emulsionspolymerisat keine ionischen Gruppen enthält.

## Claims

1. A process for coating drug forms by application of an aqueous dispersion which contains as binder a redispersed latex of a physiologically acceptable emulsion polymer onto the drug form and evaporating the water at a temperature at which the latex particles form a film, which comprises employing a polymer dispersion which has been prepared by emulsion polymerization of one part by weight of at least one ethylenically unsaturated polymerizable monomer, by means of initiators which provide free radicals, in the presence of from 0.1 to 2 parts by weight of saccharified starch of average molecular weight 2,500 - 25,000, and from 0 to 5% by weight, based on the monomer or monomers, of a surfactant, with or without further conventional additives, drying to a powder and redispersing in water.

2. A powdered mixture, which is redispersible in cold water without further aids, for the preparation of an aqueous coating agent for drug forms, which mixture contains from 10 to 100% by weight of an emulsion polymer from 1 part by weight of at least one ethylenically unsaturated polymerizable monomer which has been polymerized in the presence of from 0.1 to 2 parts by weight of saccharified starch of average molecular weight 2,500 - 25,000.

3. A powdered mixture as claimed in claim 2, wherein the emulsion polymer contains no ionic groups.

## Revendications

1. Procédé pour revêtir des médicaments façonnés par application sur ceux-ci d'une dispersion aqueuse qui contient comme liant un latex redispersé d'un polymérisat en émulsion sans inconvénient physiologique, et évaporation de l'eau à une température à laquelle les particules de latex se déposent en film, caractérisé par le fait que l'on part d'une dispersion de polymérisat qui a été préparée par polymérisation en émulsion d'une partie en poids d'au moins un monomère polymérisable insaturé éthyléniquement, au moyen d'initiateurs fournissant des radicaux libres en présence de 0,1 à 2 parties en poids d'amidon sucré de poids moléculaire moyen de 2500 à 25000, 0 à 5 % en poids, rapportés au monomère ou aux monomères, d'un produit à activité interfaciale et éventuellement d'autres additifs usuels, par mise en poudre par séchage et par redispersion dans l'eau.

2. Mélange pulvérulent, redispersable dans l'eau froide, sans autres auxiliaires, pour la préparation d'un revêtement aqueux pour médicament façonné, caractérisé par le fait qu'il contient 10 à 100 % en poids d'un polymérisat en émulsion de 1 partie en poids d'au moins un monomère polymérisable insaturé éthyléniquement, qui a été polymérisé en présence de 0,1 à 2 parties en poids d'amidon sucré d'un poids moléculaire moyen de 2500 à 25000.

3. Mélange pulvérulent selon la revendication 2, caractérisé par le fait que le polymérisat en émulsion ne contient aucun groupe ionique.
